# FASCICULE DE BREVET EUROPEEN

(11) **EP 1 383 761 B1**
(45) Date de publication et mention de la délivrance du brevet: **02.03.2011**
(21) Numéro de dépôt: 02727675.7
(22) Date de dépôt: 19.04.2002
(51) Int. Cl.: C07D 401/06, A61K 31/4725, A61P 9/00

(54) **Phenyl- et pyridyl-pipéridines avec une activité du tnf**
Phenyl- und pyridyl-piperidine mit tnf-wirkung
Phenyl- and pyridyl-piperidines with tnf activity

(30) Priorité: 20.04.2001 FR 0105361
(43) Date de publication de la demande: 28.01.2004
(73) Titulaire: Sanofi-Aventis, 75013 Paris (FR)
(72) Inventeur: BARONI, Marco, I-20010 Vanzago-Milano (IT); BOURRIE, Bernard, F-34980 Saint Gély du Fesc (FR); CASELLAS, Pierre, F-34090 Montpellier (FR)
(74) Mandataire: Varady, Peter
(86) Numéro de dépôt international: PCT/FR2002/001342
(87) Numéro de publication internationale: WO 2002/085887

(56) Documents cités:
- FR-A- 2 477 542
- DATABASE CA [en ligne] CHEMICAL ABSTRACTS SERVICE, COLUMBUS, OHIO, US; DUKIC, SLADJANA ET AL: "Synthesis and dopaminergic properties of 3- and 4-substituted 1-{2-[5-(1H-benzimidazole-2-thione)]ethyl} piperidines and related compounds" retrieved from STN Database accession no. 126:312189 XP002189144 & ARCH. PHARM. (WEINHEIM, GER.) (1997), 330(1/2), 25-28,
- DATABASE CA [en ligne] CHEMICAL ABSTRACTS SERVICE, COLUMBUS, OHIO, US; BOURRIE, BERNARD ET AL: "The neuroprotective agent SR 57746A abrogates experimental autoimmune encephalomyelitis and impairs associated blood-brain barrier disruption: implications for multiple sclerosis treatment" retrieved from STN Database accession no. 132:30802 XP002142809 & PROC. NATL. ACAD. SCI. U. S. A. (1999), 96(22), 12855-12859,

## Description

La présente invention concerne de nouvelles phényl- et pyridyl-pipéridines, les compositions pharmaceutiques les contenant et un procédé pour leur préparation.

US 5,118,691 et US 5,620,988 décrivent des tétrahydro-pyridines, substituées par un radical quinolyl-3-alkyle, montrant une activité dopaminergique.

FR 2477542 décrit des pipéridines et des pipérazines substituées par des carbostyriles, ayant une activité principalement antihistaminique.

Dukic, S. et al. (Arch. Pharm., 1997, 330(1/2): 25-28) décrivent des phényl-pipéridines portant un substituant benzimidazol-5-yl-éthyle, montrant une activité dopaminergique.

Bourrie, B et al. (Proc. Natl. Acad. Sci. USA (1999), 96(22), 12855-12859 décrivent une tétrahydropyridine substituée, montrant une activité TNF. Il a été maintenant trouvé que certaines phényl- et pyridyl-pipéridines, substituées par des radicaux hétérocycliques, possèdent une puissante activité vis-à-vis de la modulation du TNF-alpha (de l'anglais Tumour Necrosis Factor).

Le TNF-alpha est une cytokine qui a récemment suscité de l'intérêt en tant que médiateur de l'immunité, de l'inflammation, de la prolifération cellulaire, de la fibrose etc. Ce médiateur est copieusement présent dans le tissu synovial enflammé et exerce un rôle important dans la pathogenèse de l'autoimmunité (Annu. Rep. Med. Chem., 1997, 32:241-250).

Ainsi, la présente invention concerne, selon un de ses aspects, des pipéridines de formule (I) : 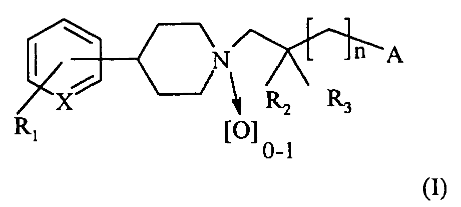 dans laquelle
- X: représente N ou CH;
- R₁: représente un atome d'hydrogène ou d'halogène ou un groupe CF₃ ;
- R₂ et R₃: représentent indépendamment un atome d'hydrogène ou un groupe méthyle ;
- n: est 0 ou 1 ;
- A: représente un groupe hétérocyclique condensé de formule (w) 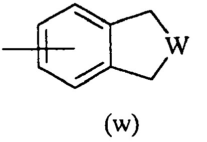
où W complète
- soit un noyau à 6 atomes, aromatique ou saturé, contenant 1 ou 2 atomes d'azote, le noyau pouvant être substitué par un ou deux atomes d'halogène, un ou deux groupes (C₁-C₄) alkyle, (C₁-C₄) alcoxy ou CF₃ ;
- soit un noyau à 5 atomes, aromatique ou saturé, contenant un atome d'azote, d'oxygène ou de soufre, le noyau pouvant être substitué par un ou deux groupes (C₁-C₄) alkyle ;
ainsi que leurs N-oxydes et leurs sels ou solvates,

Dans la présente description, le terme "(C₁-C₄)alkyle" désigne un radical monovalent d'un hydrocarbure en C₁-C₄ saturé à chaîne droite ou ramifiée et le terme "(C₁-C₄)alcoxy" désigne un radical monovalent d'un hydrocarbure en C₁-C₄ saturé à chaîne droite ou ramifiée lié par l'intermédiaire d'un atome d'oxygène.

Dans la présente description, le terme "halogène" désigne un atome choisi parmi le chlore, le brome, le iode et le fluor.

Des composés préférés sont ceux où n est zéro.

D'autres composés préférés sont ceux où R₂ et R₃ sont chacun H.

D'autres composés préférés sont ceux où R₁ est un groupe CF₃.

D'autres composés préférés sont ceux où R₁ est un atome de fluor.

D'autres composés préférés sont ceux où X est CH et R₁ est dans la position 2 ou 3 du benzène.

D'autres composés préférés sont ceux où X est CH et R₁ est un groupe CF₃.

D'autres composés préférés sont ceux où X est un atome d'azote et la pyridine est substituée dans les positions 2,6.

Des composés particulièrement préférés sont les composés de formule (I) où A est choisi parmi la quinoléine, l'isoquinoléine, la quinoxaline, la cinnoline et la phtalazine, éventuellement substituées par un ou deux groupes (C₁-C₄) alkyle.

Des composés particulièrement préférés sont les composés de formule (I) où A est choisi parmi l'indole, le benzothiophène et le benzofurane.

Selon un aspect préféré, l'invention a pour objet les composés de formule (I) où A est un groupe de formule (a) ou (b) : 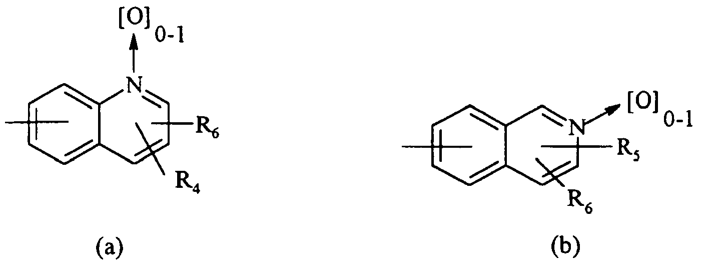 où
- R₄: représente un atome d'hydrogène ou d'halogène, un groupe (C₁-C₄) alkyle, un groupe CF₃;
- R₅: représente un atome d'hydrogène ou d'halogène, un groupe (C₁-C₄)alcoxy, un groupe (C₁-C₄)alkyle ou un groupe CF₃ ;
- R₆: représente un atome d'hydrogène, un groupe (C₁-C₄)alkyle ou un groupe (C₁-C₄)alcoxy ;
ainsi que leurs sels ou solvates.

Des composés particulièrement préférés sont les composés de formule (I) où A est un groupe de formule (a) ou (b) et R₄, R₅, et R₆ sont des atomes d'hydrogène.

Selon la présente invention, les composés de formule (I) peuvent exister comme dérivés N-oxydes. Comme indiqué dans la formule ci-dessus, les composés de formule (I) peuvent notamment porter le groupe N-oxyde sur la pipéridine ou bien sur les azotes des groupes (w), (a) et (b), ou bien tous les azotes présents peuvent être simultanément oxydés.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Les stéréoisomères optiquement purs, ainsi que les mélanges d'isomères des composés de formule (I), dus au carbone asymétrique, quand l'un de R₂ et R₃ est un méthyle et l'autre un hydrogène, dans une proportion quelconque, font partie de la présente invention.

Les composés de formule (I) peuvent être préparés par une réaction de condensation/réduction à partir d'un composé de formule (II) : 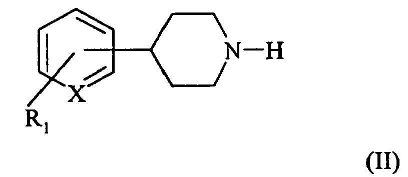 dans laquelle X et R₁ sont tels que définis ci-dessus, avec un aldéhyde de formule (III) : 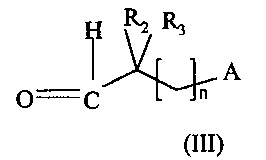 dans laquelle R₂, R₃, n et A sont tels que définis précédemment, isolement du composé de formule (I) et transformation éventuelle en un de ses sels ou solvates ou dans ses dérivés N-oxydes.

La réaction de condensation/réduction est conduite en mélangeant les composés de départ (II) et (III) dans un solvant organique tel qu'un alcool tel que par exemple le méthanol, en milieu acide, en présence d'un agent de réduction tel que le cyano-borohydrure de sodium, selon les méthodes conventionnelles.

Si l'on souhaite, on peux également utiliser, au lieu des pipéridines de départ (II), les dérivés correspondants 1,2,3,6-tétrahydro-pyridiniques et réduire la double liaison après la condensation selon des méthodes bien connues.

Alternativement, les composés de formule (I) peuvent être préparés par un procédé qui prévoit
(a) de faire réagir le composé de formule (II) : 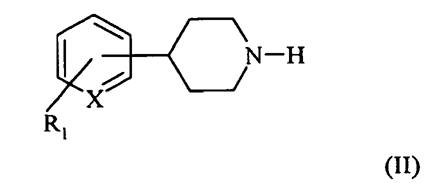 dans laquelle X et R₁ sont définis comme précédemment, avec un dérivé fonctionnel de l'acide de formule (IV) : 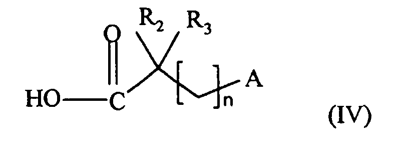 dans laquelle R₂, R₃, n et A sont tels que définis ci-dessus,
(b) de réduire le groupe carbonyle du composé de formule (V) ainsi obtenu: 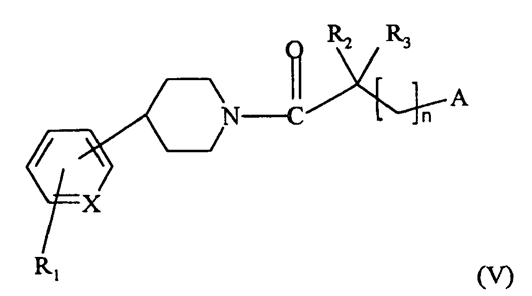
(c) d'isoler le composé de formule (I) ainsi obtenu et, éventuellement le transformer en l'un de ses sels ou solvates ou dans ses dérivés N-oxyde.

La réaction de l'étape (a) peut être convenablement conduite dans un solvant organique à une température comprise entre -10°C et la température de reflux du mélange réactionnel.

Il peut être préférable de réaliser la réaction à froid lorsqu'elle est exothermique, comme dans le cas où on utilise le chlorure en tant que dérivé fonctionnel de l'acide de formule (IV).

Comme dérivé fonctionnel approprié de l'acide de formule (IV), on peut utiliser l'acide libre, éventuellement activé (par exemple avec le BOP = tri(diméthylamino)benzotriazol-1-yloxyphosphonium hexafluorophosphate), un anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

Comme solvant de réaction, on utilise de préférence un solvant halogéné tel que le chlorure de méthylène, le dichloroéthane, le 1,1,1-trichloroéthane, le chloroforme et similaires, mais aussi d'autres solvants organiques compatibles avec les réactifs employés, par exemple le dioxane, le tétrahydrofurane ou un hydrocarbure tel que l'hexane, ou encore le diméthylformamide (DMF), peuvent être également employés.

La réaction peut être convenablement conduite en présence d'un accepteur de protons, par exemple d'un carbonate alcalin ou d'une amine tertiaire telle que la triéthylamine (TEA).

La réduction de l'étape (b) peut être convenablement réalisée par des agents de réduction appropriés tels que les complexes du borane, par exemple le diméthylsulfure de borane ([CH₃]₂S-BH₃), les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend, dans la présente invention, un solvant qui n'interfère pas avec la réaction. De tels solvants sont par exemple les éthers, tel que l'éther diéthylique, le tétrahydrofurane (THF), le dioxane ou le 1,2-diméthoxyéthane.

Selon un mode opérationnel préférentiel, on opère avec le diméthylsulfure de borane utilisé en excès par rapport au composé (III) de départ, à la température de reflux éventuellement sous atmosphère inerte. La réduction est normalement terminée après quelques heures.

Il a été remarqué que pour certains produits, en particulier lorsque W complète un noyau aromatique à 6 atomes contenant 1 ou 2 atomes d'azote, la réaction de l'étape (b) ci-dessus peut provoquer aussi la réduction dudit noyau A, notamment dans la partie complétée par W. Par conséquant, si l'on ne souhaite pas obtenir la réduction partielle du noyau A, il est alors convenable de conduire la synthèse de tels composés selon la réaction de condensation/réduction décrite ci-dessus, en utilisant comme agent de réduction le cyano-borohydrure de sodium de façon à sauvegarder les noyaux aromariques.

Il est également possibile de synthétiser les produits de formule (I) où A est un groupe (a) ou (b) par réduction des dérivés correspondants 1,2,3,6-tétrahydropyridiniques qui sont décrits dans WO01/29026, dans les condition de réduction décrites ci-dessus.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu est isolé selon les techniques usuelles et éventuellement transformé en un de ses sels ou solvates ou dans ses dérivés N-oxyde.

Les composés de départ de formule (II), (III) et (IV) sont connus ou bien ils peuvent être préparés de façon analogue aux composés connus. De tels produits sont par exemple décrits dans WO 9701536 ; J.Am. Chem. Soc.,1948, 70:2843-2847 ; J. Med. Chem., 1997,40 (7):1049.

Les composé de formule (III) peuvent par exemple être préparés en chauffant le trifluorométhylsulfonyl dérivé (autrement dit « triflate ») du composé de formule A-OH avec du N,N-dialkyléthanolamine vinyléther en présence d'un catalyseur de palladium et d'une base forte telle que par exemple la triéthylamine et en faisant réagir l'intermédiaire ainsi obtenu avec de l'acide sulfurique concentré, selon les procédures usuelles. Des exemples d'un tel procédé sont rapportés dans la partie expérimentale. Alternativement les aldéhydes de formule (III) peuvent être préparés par réduction des correspondants acides de formule (IV) selon les méthodes bien connues.

Les composés de formule (I) portant un groupe N-oxyde sur les atomes d'azote des groupes (w), (a) et (b) peuvent être préparés à partir des dérivés N-oxyde des composés de formule (III) ou (IV).

Les composés de formule (I) portant un groupe N-oxyde sur l'atome d'azote de la pipéridine peuvent être préparés par oxydation des composé de formule (I) correspondant. Dans ce cas, le composé de formule (I) tel qu'obtenu par les synthèses ci-dessus, est soumis à une réaction d'oxydation selon les méthodes conventionnelles, par exemple à une réaction avec de l'acide m-chloro-perbenzoïque dans un solvant convenable et isolé selon les techniques usuelles bien connues à l'homme du métier.

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055 :B5, Sigma, St Louis, Mo).

Les produits à tester sont administrés par voie orale à des groupes de 5 souris Balb/c femelles (Charles River, France) âgées de 7 à 8 semaines. Une heure après, le LPS est administré par voie intraveineuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, des composés représentatifs de l'invention se sont montrés très actifs, en inhibant la synthèse du TNF-α même à doses très faibles.

Grâce à cette activité et à leur faible toxicité, les composés de formule (I) et ses sels ou solvates peuvent bien être utilisés dans le traitement des maladies liées à des troubles immunitaires et inflammatoires ou comme analgésiques. Notamment les composés de formule (I) peuvent être utilisés pour traiter l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations.

Les composés de formule (I) et leurs sels et solvates pharmaceutiquement acceptables sont de préférence administrés par voie orale.

Dans les compositions pharmaceutiques de la présente invention par voie orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,001 à 100 mg, mieux de 0,01 à 50 mg, de préférence de 0,1 à 20 mg de principe actif, avantageusement de 0,5 à 10 mg.

Selon un autre de ses aspects, la présente invention concerne une association comprenant un composé de formule (I) ou l'un de ses sels ou solvates pharmaceutiquement acceptables, et au moins un composé choisi parmi les agents immunosuppresseurs, tel que l'interféron bêta-1b; l'hormone adrénocorticotrope; les glucocorticoïdes tels que la prednisone ou la méthylprednisolone; les inhibiteurs de l'interleukine-1.

Plus particulièrement, l'invention concerne une association comprenant un composé de formule (I), ou l'un de ses sels ou solvates pharmaceutiquement acceptables et au moins un composé choisi parmi le roquinimex (1,2-dihydro-4-hydroxy-N,1-diméthyl-2-oxo-3-quinolinecarboxanilide), le myloran (produit de la société Autoimmune contenant de la myéline bovine), l'antegren (anticorps humain monoclonal des sociétés Elan/Athena Neurosciences) l'interféron bêta-1b recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-aminopyridine).

Selon un autre de ses aspects, l'invention concerne une méthode de traitement des maladies liées à des troubles immunitaires et inflammatoires ainsi que dans le traitement de la douleur, notamment l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I) ou de l'un de ses sels ou solvates pharmaceutiquement acceptables, seul ou en association avec d'autres principes actifs.

Les exemples qui suivent illustrent l'invention.

### PREPARATION

### 7-isoquinolyl-acétaldéhyde

On refroidit à 0°C 1,5 g (0,0103 mole) de 7-hydroxy-isoquinoléine et 5,3 ml de pyridine. On y ajoute goutte à goutte 1,86 ml d'anhydride triflique. On agite pendant 1 heure à 0°C et après 2 heures à la température ambiante. On verse dans un mélange eau/glace, on extrait à l'acétate d'éthyle, on sèche la phase organique et on évapore le solvant sous pression réduite. On purifie le brut par chromatographie sur colonne de gel de silice en éluant par un mélange cyclohexane/acétate d'éthyle = 7/3. On obtient la 7-hydroxy-isoquinoléine trifluorométhanesulfonate sous forme d'huile. On mélange sous argon 1,65 g de ce produit avec 27,5 ml de diméthylformamide anhydre, 41 mg de acétate de palladium, 1,65 ml de triéthylamine anhydre, 1,38 g de N,N-diéthyléthanolamine vinyléther. On chauffe à 80°C pendant 36 heures. On verse dans un mélange eau/acétate d'éthyle, on sépare les deux phases, on lave la phase organique à l'eau on sèche et on évapore le solvant sous pression réduite. On purifie le résidu par chromatographie sur colonne de gel de silice en éluant par un mélange acétate d'éthyle/méthanol = 9/1. On obtient la 2-[2-(7-isoquinolyl)éthenyl)oxy)]-N,N-diéthyl-1-éthanamine. On traite 1,5 g de ce produit avec 130 ml d'eau et 13 ml d'acide sulfurique à 96%. On chauffe pendant 4,5 heures à 60°C, on verse dans de la glace, on y ajoute une solution aqueuse saturée en NaHCO₃ et on extrait à l'acétate d'éthyle. On sèche la phase organique et on évapore le solvant sous pression réduite. On obtient le composé du titre.

### EXEMPLE 1

### 7-(2-(4-(3-trifluorométhyl-phényl)-pipéridino)éthyl)-isoquinoléine et son dichlorhydrate dihydraté.

### 1a. 7-(2-(4-(3-trifluorométhyl-phényl)-1,2,3,6-tétrahydro-pyrid-1-yl)éthyl)-isoquinoléine et son dichlorhydrate dihydraté.

On mélange 1,75 g (0,0077 mole) de 4-(3-trifluorométhyl-phényl)-1,2,3,6-tétrahydro-pyridine, 30 ml de méthanol, 1,15 ml d'acide acétique glacial, 0,73 g d'acétate d'éthyle anhydre. On refroidit jusqu'à 0-5°C et on y ajoute 1,14 g de 7-isoquinolylacétaldéhyde (telle qu'obtenu selon la Préparation et avec précaution, 1,1 g (0,0175 mole) de cyanoborohydure de sodium. On agite pendant 1,5 heure à 0-5 °C et ensuite une nuit à la température ambiante. On ajoute 7 ml d'acide chlorhydrique concentré, on agite pendant 10 minutes, on évapore le solvant sous pression réduite, on reprend le résidu avec un mélange acétate d'éthyle/NH₄OH dilué. On sèche la phase organique sur du sulfate de sodium, on filtre et on évapore le solvant. On purifie le résidu sur colonne de gel de silice en éluant par un mélange acétate d'éthyle/méthanol = 9/1. On obtient le composé du titre. On prépare le dichlorhydrate à l'aide d'une solution d'isopropanol saturée en acide chlorhydrique. On obtient 1,1 g de produit.
P.f. (dichlorhydrate dihydraté) 230-233°C.

### 1b. 7-(2-(4-(3-trifluorométhyl-phényl)-pipéridino)éthyl)-isoquinoléine et son dichlorhydrate dihydraté.

On dissout 220 mg du produit de l'étape précédente dans 20 ml d'éthanol, on y ajoute 30 mg de Pd/C à 10% et on hydrogène à 45° C, à la pression atmosphérique pendant 6 heures. On filtre le catalyseur et on évapore le solvant sous pression réduite. On cristallise le résidu dans de l'isopropanol et on obtient 150 mg du composé du titre.
P.f. (dichlorhydrate dihydraté) 223-225°C.

### EXEMPLE 2

### 6-(2-(4-(3-trifluorométhyl-phényl)-pipéridino)éthyl)-1,2,3,4-tétrahydro-isoquinoléine et son dichlorhydrate.

On dissout 400 mg (0,8 mmole) de chlorhydrate de 6-(2-(4-(3-trifluorométhyl-phényl)-tétrahydro-pyrid-1-yl)éthyl)-isoquinoléine (préparé selon les procédures décrites dans WO01/29026) dans 40 ml d'éthanol à 90% et on y ajoute 40 mg de Pd/C à 10%. On hydrogène le mélange à la pression atmosphérique et à la température de 45°C pendant 4 heures. On filtre ensuite le catalyseur et on évapore le solvant en obtenant le produit du titre qu'on crystallise dans de l'isopropanol. On filtre le produit sous forme de solide blanc.
P.f. 299-300°C

### EXEMPLE 3

### 6-(2-(4-(3-trifluorométhyl-phényl)-pipéridino)éthyl)-1,2,3,4-tétrahydro-quinoxaline dichlorhydrate.

### 3a. 2-(quinoxalin-6-yl)-1-(4-(3-trifluorométhyl-phényl)-pipéridino)-éthanone.

On mélange 0,48 g (2,55 mmole) d'acide quinoxalin-6-yl-acétique dans 25 ml de DMF et on y ajoute 1,07 ml de TEA, 0,68 g (2,55 mmole) de chlorhydrate de 4-(3-trifluorométhyl-phényl)-pipéridine et 1,13 mg de BOP. On chauffe à la température ambiante pendant 1 nuit, on ajoute 200 ml d'eau et on extrait à l'acétate d'éthyle. On sèche la phase organique sur du sulfate de sodium, on filtre et on évapore le solvant sous pression réduite. On obtient ainsi une huile foncée qu'on purifie par chromatographie flash sur colonne de gel de silice en éluant avec de l'acétate d'éthyle. On obtient 0,76 g du produit du titre sous forme d'huile jaune.

### 3b. 6-(2-(4-(3-trifluorométhyl-phényl)-pipéridino)éthyl)-1,2,3,4-tétrahydro-quinoxaline et son dichlorhydrate.

On dissout sous courant d'azote 0,75 g du produit de l'étape précédente dans 25 ml de THF et on refroidit à 0°C. On y ajoute 3,76 ml de LiAlH₄ (solution 1M dans THF) en portions. On agite une nuit à la température ambiante et on y ajoute après de l'eau pour terminer la réaction. On extrait à l'acétate d'éthyle, on sèche la phase organique sur du sulfate de sodium, on filtre et on évapore le solvant sous pression réduite. On obtient une huile foncée qu'on purifie par chromatographie flash sur colonne de gel de silice en éluant avec un mélange méthanol/acétate d'éthyle= 1/1. On prépare le dichlorhydrate avec de l'isopropanol saturé en acide chlorhydrique. On évapore le solvant et l'acide chlorhydrique en excès sous pression réduite et on obtient ainsi le produit du titre sous forme de solide vitreux.
P.f. 85-87°C

### EXEMPLES 4-6

En opérant selon le mode opératoire décrit dans l'Exemple 3, on prépare les composés de formule (I) suivants:

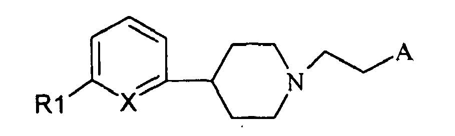

| Ex. | R1 | X | A | P.f. |
|---|---|---|---|---|
| 4 | CF₃ | CH | 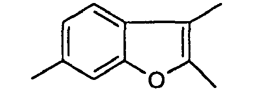 | 158-161°C (oxalate) |
| 5 | CF₃ | CH | 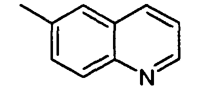 | 217-221°C (chlorhydrate) |
| 6 | CF₃ | CH | 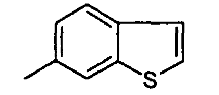 | 150-154°C (oxalate) |

| | | | | |
|---|---|---|---|---|
| Note: Dans l'Exemple 5 la réduction de l'étape (b) a été conduite avec du diméthylsulfure de borane ([CH₃]₂S-BH₃) au lieu du LiAlH₄. | | | | |

### EXEMPLES 7-9

En opérant selon le mode opératoire décrit dans l'Exemple la, en utilisant les aldéhydes convanables et les dérivés de la pipéridine au lieu de la 4-(3-trifluorométhyl-phényl)-1,2,3,6-tétrahydro-pyridine on prépare les composés de formule (I) suivants:

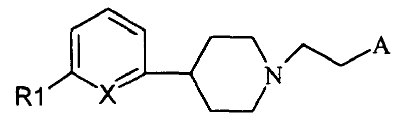

| Ex. | R1 | X | A | P.f. |
|---|---|---|---|---|
| 7 | H | CH | 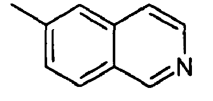 | 109-111°C (base libre) |
| 8 | CF₃ | CH | 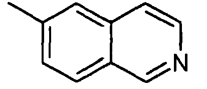 | 105-108°C (oxalate) |
| 9 | CF₃ | N | 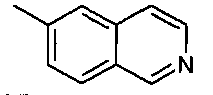 | 87-90°C (oxalate) |

## Revendications

1. Composé de formule (I) : 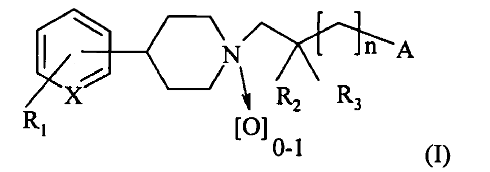 dans laquelle
X représente N ou CH;
R₁ représente un atome d'hydrogène ou d'halogène ou un groupe CF₃ ;
R₂ et R₃ représentent indépendamment un atome d'hydrogène ou un groupe méthyle ;
n est 0 ou 1 ;
A représente un groupe hétérocyclique condensé de formule (w) 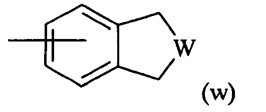
où W complète
- soit un noyau à 6 atomes, aromatique ou saturé, contenant 1 ou 2 atomes d'azote, le noyau pouvant être substitué par un ou deux atomes d'halogène, un ou deux groupes (C₁-C₄) alkyle, (C₁-C₄) alcoxy ou CF₃ ;
- soit un noyau à 5 atomes, aromatique ou saturé, contenant un atome d'azote, d'oxygène ou de soufre, le noyau pouvant être substitué par un ou deux groupes (C₁-C₄) alkyle ;
ainsi que leurs N-oxydes et leurs sels ou solvates,

2. Composé selon la revendication 1 où A représente un groupe de formule (a) ou (b) 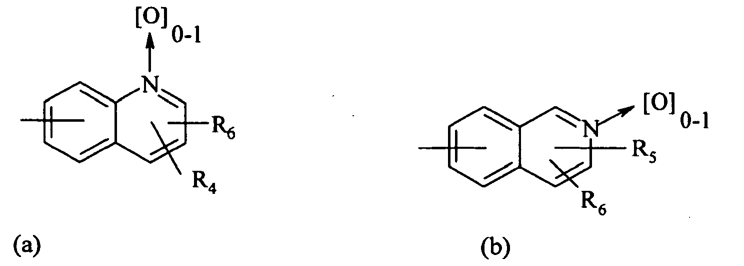 où
R₄ représente un atome d'hydrogène ou d'halogène, un groupe (C₁-C₄) alkyle, un groupe CF₃;
R₅ représente un atome d'hydrogène ou d'halogène, un groupe (C₁-C₄)alcoxy, un groupe (C₁-C₄)alkyle ou un groupe CF₃ ;
R₆ représente un atome d'hydrogène, un groupe (C₁-C₄)alkyle ou un groupe (C₁-C₄)alcoxy;
ainsi que ses sels ou solvates.

3. Composé selon la revendication 1 où A est choisi parmi le benzofurane, le benzothiophène, l'indole, la quinoxaline, la cinnoline et la phtalazine, éventuellement substitués par un ou deux groupes (C₁-C₄) alkyle.

4. Composé selon les revendications de 1 à 3 où n est zéro.

5. Composé selon les revendications 1 à 4 où R₂ et R₃ sont chacun H.

6. Composé selon les revendications de 1 à 5 où R₁ est un groupe CF₃.

7. Composé selon les revendications de 1 à 5 où R₁ est un atome de fluor.

8. Composé selon les revendications de 1 à 5 où X est CH et R₁ est dans la position 3 du benzène.

9. Composé selon les revendications de 1 à 5 où X est CH et R₁ est dans la position 2 du benzène.

10. Composé selon les revendications de 1 à 5 où X est un atome d'azote et la pyridine est substituée dans les positions 2,6.

11. Composé selon les revendications de 1 à 10 sous la forme de ses dérivés N-oxyde.

12. Composé selon la revendication 2 où R₄, R₅, et R₆ sont des atomes d'hydrogène.

13. La 7-(2-(4-(3-trifluorométhyl-phényl)-pipéridino)éthyl) isoquinoléine, ses dérivés mono-N-oxyde, bis-N-oxyde, ses sels et solvates.

14. Procédé pour la préparation des composés de formule (I) selon les revendications 1 à 13 qui comprend conduire une réaction de condensation/réduction à partir d'un composé de formule (II) : 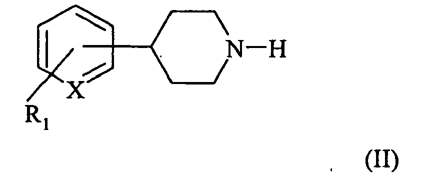 dans laquelle X et R₁ sont tels que définis dans la revendication 1, avec un aldéhyde de formule (III) : 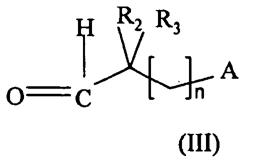 dans laquelle R₂, R₃, n et A sont tels que définis dans la revendication 1, isoler le composé de formule (I) et éventuellement le transformer en un de ses sels ou solvates ou dans ses dérivés N-oxydes.

15. Composition pharmaceutique contenant en tant que principe actif un composé de formule (I) selon les revendications de 1 à 13 ou un de ses sels ou solvates pharmaceutiquement acceptables.

16. Composition selon la revendication 15 **caractérisée en ce qu'**elle contient de 0,001 à 100 mg de principe actif.

17. Utilisation d'un composé de formule (I) selon les revendications de 1 à 13 ou d'un de ses sels ou solvates pharmaceutiquement acceptables pour la préparation de médicaments analgésiques et/ou destinés au traitement des maladies liées à des troubles immunitaires et inflammatoires.

18. Médicament contenant en tant que principe actif un composé de formule (I) selon les revendications de 1 à 13 ou un de ses sels ou solvates pharmaceutiquement acceptables.

## Claims

1. Compound of formula (I): 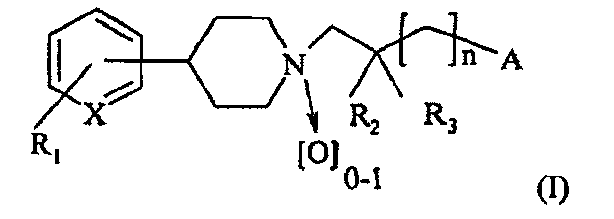 in which
X represents N or CH;
R₁ represents a hydrogen or halogen atom or a CF₃ group;
R₂ and R₃ independently represent a hydrogen atom or a methyl group;
n is 0 or 1;
A represents a fused heterocyclic group of formula (w) 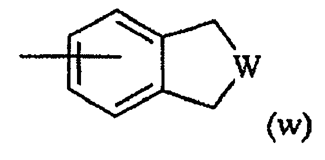
in which W completes
- either an aromatic or saturated 6-atom nucleus, containing 1 or 2 nitrogen atoms, the nucleus possibly being substituted with one or two halogen atoms or one or two groups (C₁-C₄)alkyl, (C₁-C₄)alkoxy or CF₃;
- or an aromatic or saturated 5-atom nucleus, containing a nitrogen, oxygen or sulfur atom, the nucleus possibly being substituted with one or two groups (C₁-C₄)alkyl;
and also the N-oxides thereof and the salts or solvates thereof.

2. Compound according to Claim 1, in which
A represents a group of formula (a) or (b) 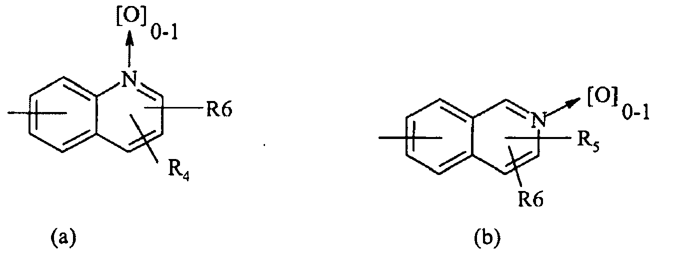 in which
R₄ represents a hydrogen or halogen atom, a (C₁-C₄)alkyl group or a CF₃ group;
R₅ represents a hydrogen or halogen atom, a (C₁-C₄)alkoxy group, a (C₁- C₄)alkyl group or a CF₃ group;
R₆ represents a hydrogen atom, a (C₁- C₄)alkyl group or a (C₁-C₄)alkoxy group;
as well as the salts or solvates thereof.

3. Compound according to Claim 1, in which A is chosen from benzofuran, benzothiophene, indole, quinoxaline, cinnoline and phthalazine, optionally substituted with one or two groups (C₁-C₄)alkyl.

4. Compound according to Claims 1 to 3, in which n is zero.

5. Compound according to Claims 1 to 4, in which R₂ and R₃ each are H.

6. Compound according to Claims 1 to 5, in which R₁ is a CF₃ group.

7. Compound according to Claims 1 to 5, in which R₁ is a fluorine atom.

8. Compound according to Claims 1 to 5, in which X is CH and R₁ is in position 3 of the benzene.

9. Compound according to Claims 1 to 5, in which X is CH and R₁ is in position 2 of the benzene.

10. Compound according to Claims 1 to 5, in which X is a nitrogen atom and the pyridine is substituted in positions 2 and 6.

11. Compound according to Claims 1 to 10, in the form of the N-oxide derivatives thereof.

12. Compound according to Claim 2, in which R₄, R₅ and R₆ are hydrogen atoms.

13. 7-(2-(4-(3-Trifluoromethylphenyl)piperidino)ethyl)isoquinoline, the mono-N-oxide and bis-N-oxide derivatives thereof and the salts and solvates thereof.

14. Process for preparing the compounds of formula (I) according to Claims 1 to 13, which comprises carrying out a coupling/reduction reaction from a compound of formula (II): 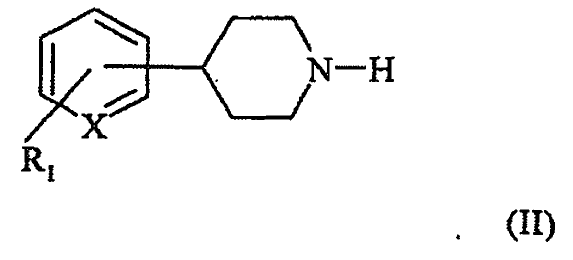 in which X and R₁ are as defined in Claim 1, with an aldehyde of formula (III): 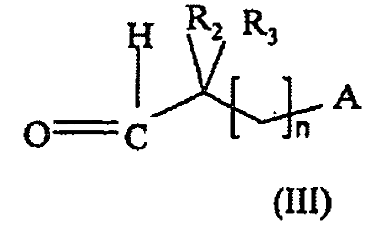 in which R₂, R₃, n and A are as defined in Claim 1, isolating the compound of formula (I) and optionally converting it into a salt or solvate thereof or an N-oxide derivative thereof.

15. Pharmaceutical composition containing, as active principle, a compound of formula (I) according to Claims 1 to 13 or a pharmaceutically acceptable salt or solvate thereof.

16. Composition according to Claim 15, **characterized in that** it contains from 0.001 to 100 mg of active principle.

17. Use of a compound of formula (I) according to Claims 1 to 13, or of a pharmaceutically acceptable salt or solvate thereof, for the preparation of analgesic medicinal products, and/or medicinal products which are intended for the treatment of diseases associated with immune and inflammatory disorders.

18. Medicament containing, as active principle, a compound of formula (I) according to Claims 1 to 13 or a pharmaceutically acceptable salt or solvate thereof.

## Patentansprüche

1. Verbindung der Formel (I): 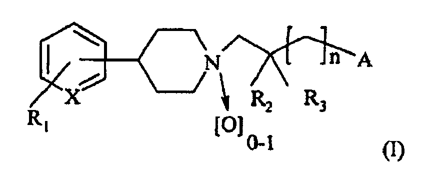 in der
X N oder CH bedeutet;
R₁ ein Wasserstoffatom oder ein Halogenatom oder eine CF₃-Gruppe bedeutet;
R₂ und R₃ unabhängig voneinander ein Wasserstoffatom oder eine Methylgruppe bedeuten;
n 0 oder 1 bedeutet;
A eine kondensierte heterocyclische Gruppe der Formel (w) 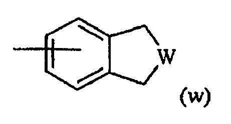
bedeutet,
in der W
- entweder einen aromatischen oder gesättigten Kern mit 6 Atomen, der 1 oder 2 Stickstoffatome enthält, vervollständigt, wobei der Kern durch ein oder zwei Halogenatome, eine oder zwei (C₁-C₄)-Alkyl-, (C₁-C₄)-Alkoxy- oder CF₃-Gruppen substituiert sein kann;
- oder einen aromatischen oder gesättigten Kern mit 5 Atomen, der ein Stickstoffatom, Sauerstoffatom oder Schwefelatom enthält, vervollständigt, wobei der Kern durch eine oder zwei (C₁-C₄)-Alkylgruppen substituiert sein kann;
sowie ihre N-Oxide und ihre Salze oder Solvate.

2. Verbindung nach Anspruch 1, in der A eine Gruppe der Formel (a) oder (b) bedeutet, 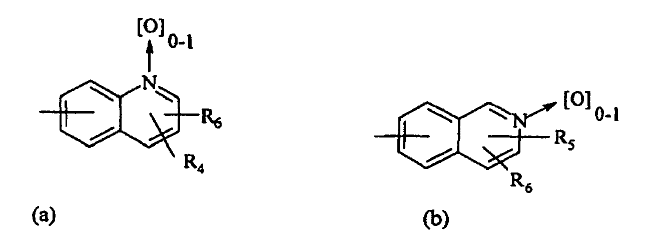 in der
R₄ ein Wasserstoffatom oder ein Halogenatom, eine (C₁-C₄)-Alkylgruppe oder eine CF₃- Gruppe bedeutet;
R₅ ein Wasserstoffatom oder ein Halogenatom, eine (C₁-C₄)-Alkoxygruppe, eine (C₁-C₄)- Alkylgruppe oder eine CF₃-Gruppe bedeutet;
R₆ ein Wasserstoffatom, eine (C₁-C₄)- Alkylgruppe oder eine (C₁-C₄)-Alkoxygruppe bedeutet;
sowie ihre Salze oder Solvate.

3. Verbindung nach Anspruch 1, in der A aus der Reihe Benzofuran, Benzothiophen, Indol, Chinoxalin, Cinnolin und Phthalazin, die gegebenenfalls durch eine oder zwei (C₁-C₄)-Alkylgruppen substituiert sind, ausgewählt ist.

4. Verbindung nach den Ansprüchen 1 bis 3, in der n null bedeutet.

5. Verbindung nach den Ansprüchen 1 bis 4, in der R₂ und R₃ jeweils H bedeuten.

6. Verbindung nach den Ansprüchen 1 bis 5, in der R₁ eine CF₃-Gruppe bedeutet.

7. Verbindung nach den Ansprüchen 1 bis 5, in der R₁ ein Fluoratom bedeutet.

8. Verbindung nach den Ansprüchen 1 bis 5, in der X CH bedeutet und R₁ sich in der 3-Position des Benzols befindet.

9. Verbindung nach den Ansprüchen 1 bis 5, in der X CH bedeutet und R₁ sich in der 2-Position des Benzols befindet.

10. Verbindung nach den Ansprüchen 1 bis 5, in der X ein Stickstoffatom bedeutet und das Pyridin in den Positionen 2,6 substituiert ist.

11. Verbindung nach den Ansprüchen 1 bis 10 in Form ihrer N-Oxidderivate.

12. Verbindung nach Anspruch 2, in der R₄, R₅ und R₆ Wasserstoffatome bedeuten.

13. 7-(2-(4-(3-Trifluoromethylphenyl)piperidino) ethyl) isochinolin, seine Mono-N-oxidderivate, Bis-N-oxidderivate, seine Salze und Solvate.

14. Verfahren zur Herstellung von Verbindungen der Formel (I) nach den Ansprüche 1 bis 13, bei dem man eine Verbindung der Formel (II): 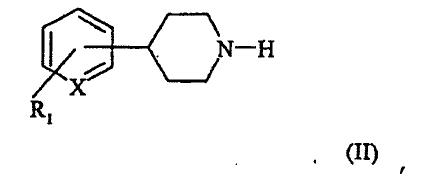 in der X und R₁ wie in Anspruch 1 definiert sind, mit einem Aldehyd der Formel (III): 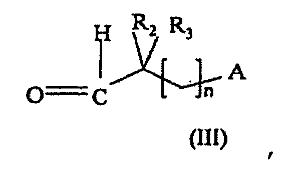 in der R₂, R₃, n und A wie in Anspruch 1 definiert sind, kondensiert/reduziert, die Verbindung der Formel (I) isoliert und sie gegebenenfalls in eines ihrer Salze oder Solvate oder in ihre N-Oxidderivate umwandelt.

15. Pharmazeutische Zusammensetzung, die als Wirkstoff eine Verbindung der Formel (I) nach den Ansprüchen 1 bis 13 oder eines ihrer pharmazeutisch unbedenklichen Salze oder Solvate enthält.

16. Zusammensetzung nach Anspruch 15, **dadurch gekennzeichnet, dass** sie 0,001 bis 100 mg Wirkstoff enthält.

17. Verwendung einer Verbindung der Formel (I) nach den Ansprüchen 1 bis 13 oder eines ihrer pharmazeutisch unbedenklichen Salze oder Solvate für die Herstellung von schmerzstillenden Arzneimitteln und/oder für die Behandlung von mit Immunstörungen und entzündlichen Störungen assoziierten Krankheiten.

18. Arzneimittel, das eine Verbindung der Formel (I) nach den Ansprüchen 1 bis 13 oder eines ihrer pharmazeutisch unbedenklichen Salze oder Solvate als Wirkstoff enthält.
